# EUROPEAN PATENT APPLICATION

(11) **EP 0 820 703 A1**
(43) Date of publication of application: **28.01.1998**
(21) Application number: 97112421.9
(22) Date of filing: 21.07.1997
(51) Int. Cl.: A23L 1/302, A23L 1/303, A23L 1/304, A61K 9/50, A61K 9/20

(54) **Compositions for nutritional integration comprising prolonged-release hydrosoluble vitamins**

(30) Priority: 22.07.1996 IT MI961525
(71) Applicant: Valpharma S.A., 47031 Serravalle (SM)
(72) Inventor: Valducci, Roberto, 47039 Savignano Sul Rubicone, (Forli) (IT)
(74) Representative: Gervasi, Gemma, Dr.

(57) **Abstract**

The present invention regards compositions for nutritional integration comprising hydrosoluble vitamins having a prolonged-release formulation and liposoluble vitamins having a fast-release formulation, possibly mixed with mineral substances or other nutritional principles.

## Description

### Prior art

Vitamins are organic substances having various chemical structures that play a determining role in oxidative metabolism. Vitamins must be supplied from outside, principally through the diet, in that the human organism is not able to produce them, or produces insufficient quantities of them for its own requirements.

The metabolic damage deriving from an unbalanced vitamin intake is relatively frequent and can be detected clinically.

Various causes are indicated, such as the impoverishment caused by methods of intensive production and processes of refining and/or preserving food. Different preparations containing vitamins are therefore commonly administered at doses that approach those corresponding to the physiological requirement. This nutritional integration is justified in various conditions, such as that of qualitatively inadequate diets or unbalanced eating, and in periods of particular demands of the organism, such as pregnancy, breast-feeding and old age.

The importance of identifying optimal intake levels of nutritional principles is, on the other hand, highlighted by the fact that in almost all countries of the world nutritional expert committees have been set up with the aim of constantly updating the recommended levels of intake. Examples of the results of such committees are the European Recommended Dietary Allowances (EU-RDA), the U.S. Recommnended Dietary Allowances (US-RDA), and, for the Italian population, the Recommended Daily Levels of Intake of Energy and Nutrients (L.A.R.N.).

The compositions today available for nutritional integration of vitamins are aimed simply at supplying the quantities equivalent to the physiological requirement, without seeking in any way to optimize the availability and the use of vitamins by the organism.

It is important to emphasize that, apart from the obvious differences at an individual level, there exists a number of general aspects regarding the use of vitamins by the organism which are determined by the characteristics of the vitamins themselves.

In fact, hydrosoluble vitamins are rapidly eliminated by the organism, and hence there is the risk of it not maintaining over time adequate tissue levels of these vitamins.

As far as liposoluble vitamins are concerned, instead, the human organism is able to build up reserves upon which it can draw according to its own requirements.

Since the elimination of hydrosoluble vitamins is extremely rapid, with the conventional administration of high doses, high temporary concentrations are generated that are bound to be poorly used by the organism. Consequently, the compositions containing conventional-release hydrosoluble vitamins do not represent a rational nutritional integration, even though the temporary excess of the vitamins themselves does not seem to constitute any risk for the organism.

### Summary

The problems of the known art are overcome by the compositions for nutritional integration according to the present invention.

The aforesaid compositions include hydrosoluble vitamins, liposoluble vitamins, and possibly mineral substances or other nutritional principles, and are characterized by the fact that the hydrosoluble vitamins have a prolonged-release formulation and possibly in part a rapid-release formulation, and the liposoluble vitamins have a rapid-release formulation.

The aforesaid compositions enable optimal use of the daily necessary amounts of vitamins and of the possible nutritional principles associated thereto, and in particular enable a slow and gradual release of the hydrosoluble vitamins.

### Detailed description of the invention

The characteristics and advantages of the compositions for nutritional integration that include prolonged-release hydrosoluble vitamins according to the present invention shall be further illustrated in the course of the detailed description that follows.

The aforesaid compositions include hydrosoluble vitamins having a formulation that is capable of prolonged release, and liposoluble vitamins having a formulation capable of rapid release.

The aforesaid compositions may also include mineral substances, and possibly other nutritional principles.

If so desired, one part of the hydrosoluble vitamins, from 1% to 80% in weight, is present in a formulation for fast release.

Various known techniques may be employed to obtain the prolonged-release formulations.

For example, the vitamins are applied by means of an ethanol solution of shellac on inert microgranules made of starch and sucrose in a rotating pan, and the microgranules thus obtained are surface-coated with shellac or with acrylic polymers, derivatives of cellulose, hydrogenated vegetable oils, fatty acids, paraffin or mixtures of these.

The microgranules described, without the surface coating, constitute formulations for fast release.

Another technique for obtaining hydrosoluble vitamin formulations for prolonged release consists in preparing microgranules or tablets in which the vitamins are formulated with hydroxypropylmethyl cellulose.

A technique for obtaining fast-release compositions, particularly for liposoluble vitamins, consists in preparing microgranules or tablets having a lactose and starch base.

The compositions according to the present invention are made in the form of tablets, capsules or granules to be contained in bags with a unit dose of the individual vitamins, mineral substances, or other nutritional active principles possibly associated thereto, ranging from 0.25 to 5.0 times the daily officially recommended intake values.

In particular, the tablets may be of the multilayer type, in which one layer contains the hydrosoluble vitamins having prolonged release, whilst another layer contains the liposoluble vitamins having fast release.

The ratio in weight between the hydrosoluble vitamins and the liposoluble vitamins is between 1:100 and 100:1.

By means of the administration of the compositions according to the present invention, the liposoluble vitamins are made available to the organism in a time interval of less than one hour, whereas the hydrosoluble vitamins are made available in a gradual way, i.e., in time intervals of over eight hours.

The compositions according to the present invention thus solve the problem of a rational nutritional integration that enables the maintenance of an optimal trophism and optimal state of metabolic functioning and physical efficiency.

As illustrations of the invention the following examples are given.

### Example 1

Granules containing hydrosoluble vitamins having prolonged release were prepared according to the following procedure.

The components were weighed:

| | |
|---|---|
| Ascorbic acid (Vit. C) | 6000 g |
| Nicotinamide (Vit. PP) | 1000 g |
| Inert granules | 12000 g |
| Shellac | 540 g |
| Ethanol | 2200 g |

The inert granules consisted of starch and sucrose in the proportion of 20:80 in weight and had a mean diameter of approx. 0.6 mm.

The preparation was made in two steps:

### Step a)

The aforesaid inert granules were put into a rotating pan.

The vitamins and shellac were dissolved in ethanol, and the solution obtained was gradually fed into the rotating pan that contained the inert granules, at a temperature of 30°C.

With this procedure, the vitamins were applied on the said inert granules.

A sample of the composition thus obtained revealed a high rate of dissolution of the vitamins in water. In fact, after one hour, 100% of the vitamins had dissolved.

### Step b)

The granules obtained as in Step a) were coated with shellac according to the following procedure.

An alcohol solution of shellac was prepared consisting of 56 g of shellac and 84 g of ethanol.

1 kg of the granules obtained as in Step a) was put into a rotating pan, and on the granules the solution consisting of 56 g of shellac and 84 g of ethanol was gradually fed.

This operation was carried out at a temperature of 30°C.

The coating process was facilitated by the addition of talcum, in a quantity of 120 g, added at the same time as the application of the coating solution. The granules were subsequently dried in a forced-ventilation oven at 40°C for 48 hours.

The granules thus obtained underwent a dissolution test in water according to the method described as type 2 in USP XXII.

The results obtained, referring to the release of nicotinamide, are given in the following table:

| h | Dissolved vitamins % by weight |
|---|---|
| 1 | 36.3 |
| 4 | 58.0 |
| 8 | 74.4 |

### Example 2

Granules containing hydrosoluble vitamins presenting prolonged release were prepared, using the method described below.

The following components were weighed:

| | |
|---|---|
| Calcium pantothenate | 1000 g |
| Pyridoxine HCl (Vit. B6) | 252 g |
| Thiamine mononitrate(Vit. B1) | 180 g |
| Folic acid | 32 g |
| Inert granules (starch and sucrose 20/80 in weight) | 1200 g |

Using the method described in Example 1, Step a), granules were obtained which at the dissolution test in water showed a release of thiamine mononitrate and pyridoxine HCl of 100% in one hour.

One kg of said granules underwent coating with a kg of shellac in 40% ethanol solution according to the operating procedures described in Example 1, Step b).

The dissolution test in water of coated granules yielded the following results:

| h | Dissolved vitamins, % by weight | |
|---|---|---|
| | Pyridoxine | Thiamine |
| 1 | 35.2 | 33.3 |
| 4 | 49.6 | 45.4 |
| 8 | 78.3 | 74.9 |

### Example 3

Granules containing liposoluble vitamins having rapid release were prepared, using the method described below.

The following components were weighed:

| | |
|---|---|
| Vitamin E | 500 g |
| Cholecalciferol (D3) | 0.170 g |
| b-carotene | 350 g |
| Inert granules (starch and sucrose 28/80 in weight) | 500 g |
| Shellac | 120 g |

Using the method described in Example 1, Step a), granules were obtained in which the vitamins showed a release in water of 100% in one hour.

### Example 4

Capsules were prepared containing granules comprising hydrosoluble vitamins and granules comprising liposoluble vitamins, using the method described below.

The granules obtained in Examples 1, 2 and 3 were dosed and mixed with the aim of making them into capsules of gelatine with the following dosages:

| | |
|---|---|
| Thiamine | 4.5 mg |
| Pyridoxine | 6.3 mg |
| Ascorbic acid | 150.0 mg |
| Calcium pantothenate | 25 mg |
| Nicotinamide | 25 mg |
| Folic acid | 0.800 mg |
| b-carotene | 6 mg |
| Cholecalciferol | 10 mcg |

### Example 5

Tablets containing hydrosoluble vitamins having prolonged release were prepared, using the method described below.

The following components were weighed:

| | |
|---|---|
| Thiamine | 45.0 g |
| Riboflavin | 54.0 g |
| Ascorbic acid | 500.0 g |
| Biotin | 3.0 g |
| Pyridoxine HCl | 63.0 g |
| Cyanocobalamin (Vitamin B₁₂) | 0.025 mg |
| Folic acid | 8.0 g |
| Hydroxypropylmethyl cellulose | 98.0 g |

The components indicated were granulated using the wet-granulation process with the addition of ethanol and subsequently dried at 35°C in a forced-ventilation oven. On the granules obtained the thiamine titre was evaluated, which was found to be 58.3 mg/g. From these granules were prepared tablets having a mean weight of 77 mg.

From the dissolution test in water the following results were obtained:

| h | Dissolved vitamins, % by weight | | |
|---|---|---|---|
| | Thiamine | Ascorbic acid | Pyridoxine |
| 1 | 26.2 | 35.1 | 23.0 |
| 4 | 49.5 | 70.2 | 60.5 |
| 8 | 83.1 | 93.0 | 82.4 |

### Example 6

Using the same procedure described in Example 5, tablets having a weight of 81 mg were produced, obtained from a mixture having the following composition (hydrosoluble vitamins):

| | |
|---|---|
| Ascorbic acid | 500 g |
| Calcium pantothenate | 250 g |
| Hydroxypropylmethyl cellulose | 63 g |
| Ascorbic acid titre | 615 mg/g |

From the dissolution test in water the following results were obtained:

| h | Dissolved vitamins, % by weight | |
|---|---|---|
| | Ascorbic acid | Calcium pantothenate |
| 1 | 38.1 | 27.4 |
| 4 | 67.6 | 58.2 |
| 8 | 91.5 | 80.6 |

### Example 7

Using the same procedure described in Example 5, tablets having a weight of 81 mg were produced, obtained from a mixture having the following composition (hydrosoluble vitamins):

| | |
|---|---|
| Ascorbic acid | 500 g |
| Nicotinamide | 250 g |
| Hydroxypropylmethyl cellulose | 63 g |

From the dissolution test in water the following results were obtained:

| h | Dissolved vitamins, % by weight | |
|---|---|---|
| | Ascorbic acid | Nicotinamide |
| 1 | 36.3 | 21.4 |
| 4 | 68.9 | 54.7 |
| 8 | 90.4 | 90.2 |

### Example 8

Using the same procedure described in Example 5, tablets were produced, obtained from a mixture having the following composition (liposoluble vitamins):

| | |
|---|---|
| b-carotene 30% | 200 g |
| Vitamin E | 300 g |
| Lactose | 150 g |
| Starch | 200 g |
| Colloidal siliceous earth | 50 g |
| Cholecalciferol | 110 g |
| b-carotene titre | 66.66 mg/g |

Tablets having a weight of 90 mg were produced. The dissolution test *in vitro* shows that the release of the vitamins produced occurs in less than one hour:

### Example 9 (Capsules were prepared containing tablets with hydrosoluble vitamins and tablets with liposoluble vitamins)

Capsules containing tablets comprising hydrosoluble vitamins and tablets comprising liposoluble vitamins, using the method described below.

The tablets obtained in Examples 5, 6, 7 and 8 were loaded in capsules with a dosage such as to obtain the following unit composition:

| | |
|---|---|
| Thiamine | 4.50 mg |
| Riboflavin | 5.40 g |
| Pyridoxine HCl | 6.30 g |
| Cyanocobalamin (Vitamin B₁₂) | 2.5 mg |
| Ascorbic acid | 150.0 g |
| Calcium pantothenate | 25.0 mg |
| Nicotinamide | 25.0 mg |
| Folic acid | 0.800 mg |
| Biotin | 0.3 mg |
| Cholecalciferol | 10 mg |
| Vitamin E | 30 mg |
| b-carotene | 6 mg |

### Example 10

Multilayer tablets were prepared using the method described below.

### Stage a)

Using the wet-granulation technique described in Example 5, the following mixture containing hydrosoluble vitamins was granulated:

| | |
|---|---|
| Thiamine | 45.0 g |
| Riboflavin | 54.0 g |
| Ascorbic acid | 1000.0 g |
| Pyridoxine | 63.0 g |
| Biotin | 3.0 g |
| Cyanocobalamin (Vitamin B₁₂) | 0.250 mcg |
| Folic acid | 8.0 g |
| Hydroxypropylmethyl cellulose | 73.0 mg |
| Thiamine titre | 36.2 mg/g |

### Step b)

Using the technique described in Step a), the following mixture containing hydrosoluble vitamins was granulated:

| | |
|---|---|
| Ascorbic acid | 500.0 g |
| Nicotinamide | 250.0 g |
| Calcium pantothenate | 200.0 g |
| Hydroxypropylmethyl cellulose | 100.0 g |
| Ascorbic acid titre | 456.0 mg/g |

### Step c)

Using the technique described in Step a), the following mixture containing liposoluble vitamins was granulated:

| | |
|---|---|
| b-carotene 30% | 200.0 g |
| Vitamin E | 300.0 g |
| Cholecalciferol | 110.0 mg |
| Lactose | 150.0 g |
| Starch | 200.0 g |
| Aerosil | 50.0 g |

### Step d)

Using a multilayer press, Manesty layerpresse, from Steps a), b) and c) tablets were prepared with a diameter of 10.4 mm having the following composition:
Layer 1 :- 125 mg, consisting of granules of Step a), containing prolonged-release hydrosoluble vitamins;
Layer 2 :- 90 mg, consisting of granules of Step c), containing rapid-release liposoluble vitamins;
Layer 3 :- 110 mg, consisting of granules of Step b), containing prolonged-release hydrosoluble vitamins;

### Example 11

Granules for single-dose bags or for bottles with volumetric doser were prepared, using the method described below.

The components indicated below were granulated using ethanol in a wet-granulation process and subsequently dried.

| | |
|---|---|
| Thiamine | 45.0 g |
| Riboflavin | 54.0 g |
| Ascorbic acid | 1500.0 g |
| Pyridoxine | 63.0 g |
| Biotin | 3.0 g |
| Cyanocobalamin (Vitamin B₁₂) | 0.250 mg |
| Folic acid | 8.0 g |
| Nicotinamide | 250.0 g |
| Calcium pantothenate | 250.0 g |
| Hydroxypropylmethyl cellulose | 48.0 g |

The granules obtained enable a prolonged release of the vitamins.

## Claims

1. Compositions for nutritional integration comprising hydrosoluble vitamins, liposoluble vitamins, and possibly mineral substances or other nutritional principles, characterized in that the said hydrosoluble vitamins have a prolonged-release formulation and possibly in part a rapid-release formulation, and the said liposoluble vitamins have a rapid-release formulation.

2. Compositions according to Claim 1, characterized in that said hydrosoluble vitamins having a rapid-release formulation constitute from 1% to 80% by weight of the hydrosoluble vitamins.

3. Compositions according to Claim 1, characterized in that they are made in the form of tablets, capsules and granules with a unit dose of the individual vitamins, mineral substances, or other nutritional active principles possibly associated thereto, ranging from 0.25 to 5.0 times the daily officially recommended intake values.

4. Compositions according to Claim 1, characterized in that the ratio by weight between the hydrosoluble vitamins and the liposoluble vitamins is between 1:100 and 100:1.

5. Compositions according to Claim 1, characterized in that the prolonged-release vitamins are made available to the organism in a time interval of over eight hours and that the fast-release vitamins are made available in a time interval of less than one hour.
